# EUROPEAN PATENT APPLICATION

(11) **EP 1 985 618 A1**
(43) Date of publication of application: **29.10.2008**
(21) Application number: 07107075.9
(22) Date of filing: 27.04.2007
(51) Int. Cl.: C07D 513/04

(54) **Process for the preparation of brinzolamide and intermediates thereof**

(71) Applicant: Duke Chem, S. A., 08799 Olerdola Barcelona (ES)
(72) Inventor: Catasus Ajenjo, Mònica, Dr. Duke Chem, S.A., 08799, Olèrdola (Barcelona) (ES); Garcia Torres, Jasón Duke Chem, S.A., 08799, Olèrdola (Barcelona) (ES)
(74) Representative: Barlocci, Anna

(57) **Abstract**

Preparation process of brinzolamide of formula (I) comprising the protection of the secondary amine of their non sulfonamide substituted precursor; the chlorosulfonation and the subsequent amination with ammonia of the obtained intermediate; and, finally, the deprotection of the obtained sulfonamide by acid hydrolysis. The process allows to obtain brinzolamide at a industrial scale by using milder reaction conditions and with a high yield at reduced costs. Some new intermediates are used in this process.

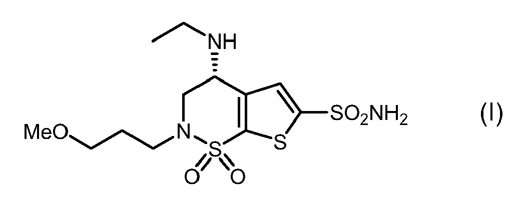

## Description

The present invention relates to a process for the preparation of an active pharmaceutical ingredient known as brinzolamide, as well as to some new intermediates useful in such preparation process.

### BACKGROUND ART

Brinzolamide is the generic name of the (4R)-4-(ethylamino)-3,4-dihydro-2-(3-methoxypropyl)-2H-thieno[3,2-e]-1,2-thiazine-6-sulfonamide 1,1-dioxide, having the structural formula (I). 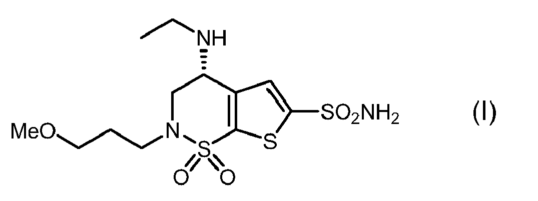 Brinzolamide is an inhibitor of carbonic anhydrase. This enzyme, found in many tissues of the body including the eye, catalyzes the reversible reaction involving the hydration of carbon dioxide and the dehydration of carbonic acid. Inhibition of carbonic anhydrase in the ciliary processes of the eye decreases aqueous humor secretion, presumably by slowing the formation of bicarbonate ions with subsequent reduction in sodium and fluid transport. The result is a reduction in intraocular pressure (IOP). Elevated intraocular pressure is a major risk factor in the pathogenesis of optic nerve damage and glaucomatous visual field loss.

Processes for the preparation of brinzolamide have been disclosed in EP 0527801-A, and in EP 0617038-A, both from Alcon Laboratories.

In EP 0527801-A, (R)-3,4-dihydro-4-alkylamino-2-substituted-2H-thieno[3,2-e]-1,2-thiazine-6-sulfonamide-1,1-dioxides, including brinzolamide, are prepared by resolution of the racemate via the di-p-toluyl-D-tartaric acid salt: 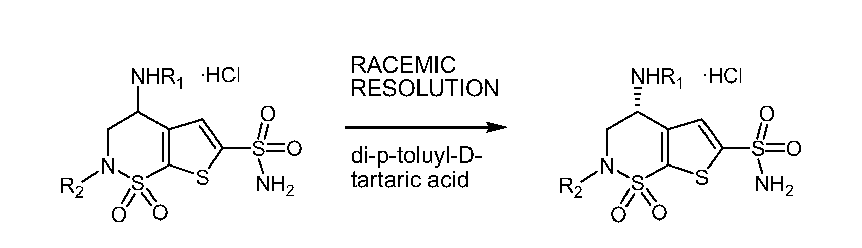 where R₁ is alkyl, and R₂ can be different radicals. In this case, the resolution of the racemic compound is carried out at the last synthetic step, what implies a considerable increase of costs.

The same document discloses a process for the preparation of the mentioned compounds from (S)-3,4-dihydro-4-hydroxy-2-substituted-2H-thieno[3,2-e]-1,2-thiazine-6-sulfonamide-1,1-dioxides by activation of the C(4)-hydroxyl group and, subsequently, displacement with the appropriate amine:

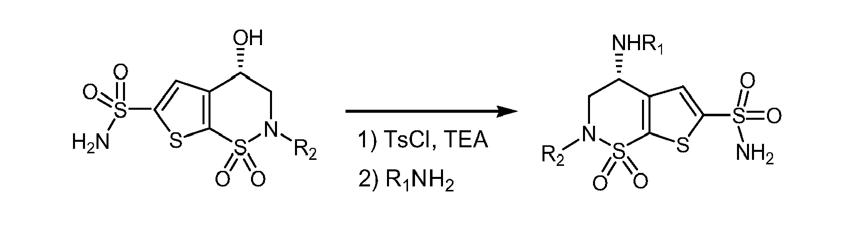

In this process, the introduction of the amine substituent at the end of the synthetic route leads to the final product with relatively low yields. Thus, for instance, in the case of brinzolamide the corresponding hydrochloride is obtained with only a 34% of yield.

In EP 0617038-A a process for the preparation of (R)-3,4-dihydro-4-alkylamino-2-substituted-2H-thieno[3,2-e]-1,2-thiazine-6-sulfonamide-1,1-dioxides from 3-acetyl-2,5-dichlorothiophene is disclosed. This process requires the use of very low temperatures and highly inflammable solvents, what makes it difficult to scale-up to an industrial level.

Despite the teaching of these prior art documents, the research of new and easy to scale up processes for the preparation of brinzolamide is still an active filed, as the industrial exploitation of the known processes is difficult or expensive. Thus, the provision of an improved process for the preparation of brinzolamide in high yields is still a matter of great interest in industry.

### SUMMARY OF THE INVENTION

Inventors have found a new process for the preparation of brinzolamide that overcomes or minimizes the drawbacks above mentioned related to the known processes to obtain the product. The new process allows obtaining brinzolamide with high yields, while using reaction conditions easy to scale-up to an industrial level, and thus reducing costs.

Accordingly, a first aspect of the present invention refers to process for the preparation of brinzolamide of formula (I), or its pharmaceutically acceptable salts, or its solvates including hydrates, 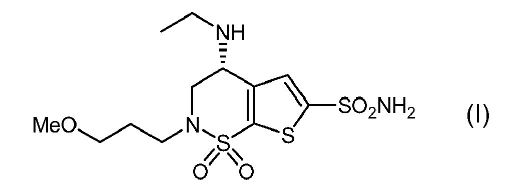 comprising a) a halosulfonation reaction of the compound of formula (IV), 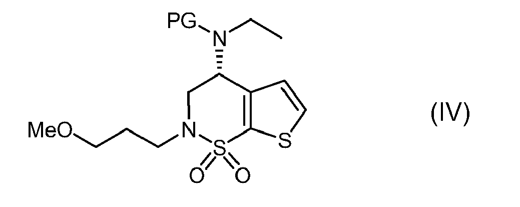 wherein PG is a protecting group, in the presence of a halosulfonating agent, to give a compound of formula (III), 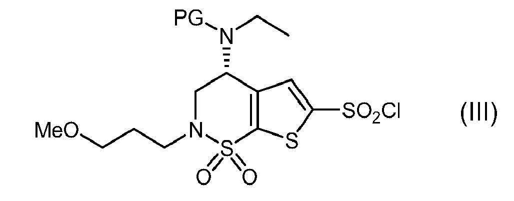 followed by the amination with an ammonia source to give a compound of formula (II), and 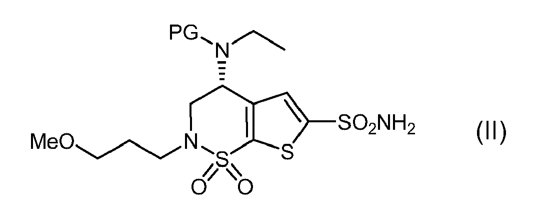 b) a deprotection reaction of the compound of formula (II).

This process differs from the above mentioned prior art processes in that the sulfonamidation reaction on C(6) is carried out over an intermediate compound with a protected alkylamine on C(4) (i.e. compound of formula (IV)) instead of over an intermediate compound with a hydroxyl or a protected hydroxyl on C(4). Inventors have found that the introduction of the alkylamine substituent at an earlier stage and before carrying out the sulfonation reaction is advantageous since the sulfonation reaction proceeds with higher yield and purity, while it is avoided to carry out the enantiomeric reaction at the last steps of the process or the resolution of the final racemic product, as opposite to the processes disclosed in the prior art to obtain brinzolamide. In particular, when the sulfonation is carried out with a mixture of an halosulfonating agent and a halogenating agent, the process is specially advantageous since milder reaction conditions are needed, shorter reaction times are employed, and the use of reactants and solvents difficult to handle at industrial level are avoided. All this results in a reduction of the cost of the global process.

According to a second aspect of the invention, new compounds of formula (V'), (V), (IV), and (II), or its pharmaceutically acceptable salts, or its solvates including hydrates, are provided as intermediates useful for the preparation of brinzolamide 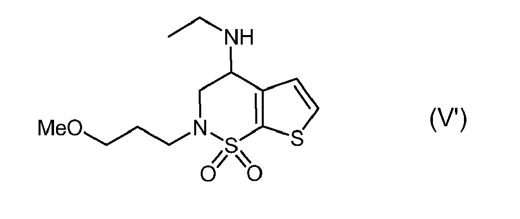 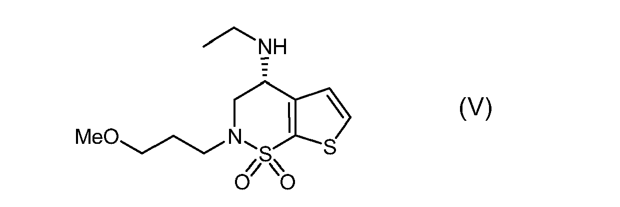 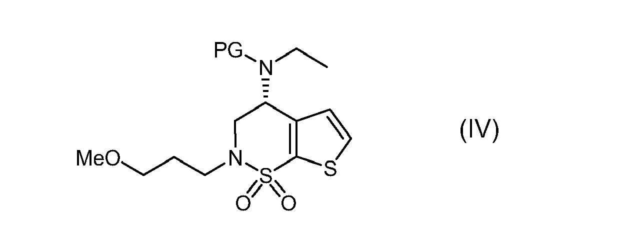 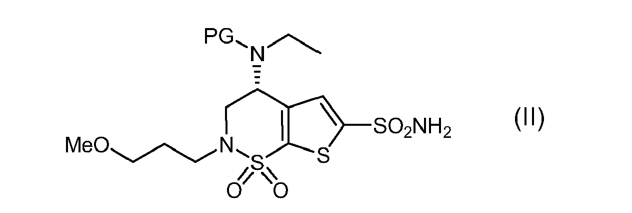 wherein PG is a protecting group.

The compounds (III), (VI), and (VII), which are further intermediates useful for the preparation of brinzolamide, also form part of the present invention.

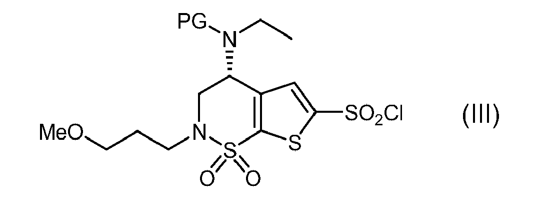

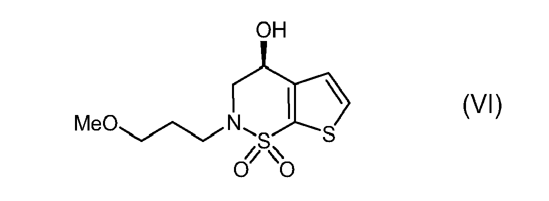

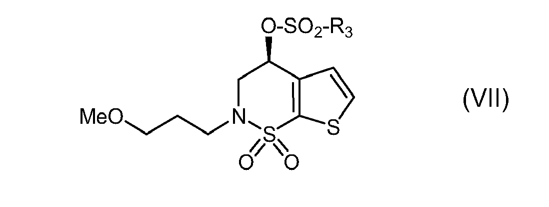

### DETAILED DESCRIPTION OF THE INVENTION

According to the first aspect of the invention, brinzolamide, or its pharmaceutically acceptable salts, or its solvates including hydrates, is prepared by a process comprising a halosulfonation reaction of the compound of formula (IV), wherein PG is a protecting group, and the subsequent amination with an ammonia source of the obtained intermediate; and, finally, the deprotection of the obtained sulfonamide of formula (II). 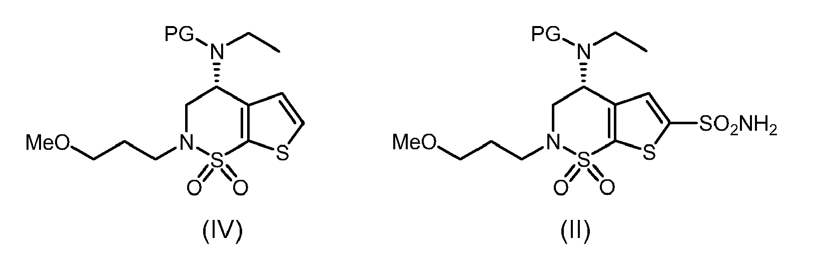 A preferred pharmaceutically acceptable salt of brinzolamide is the hydrochloride.

The process can be summarized in Scheme 1:

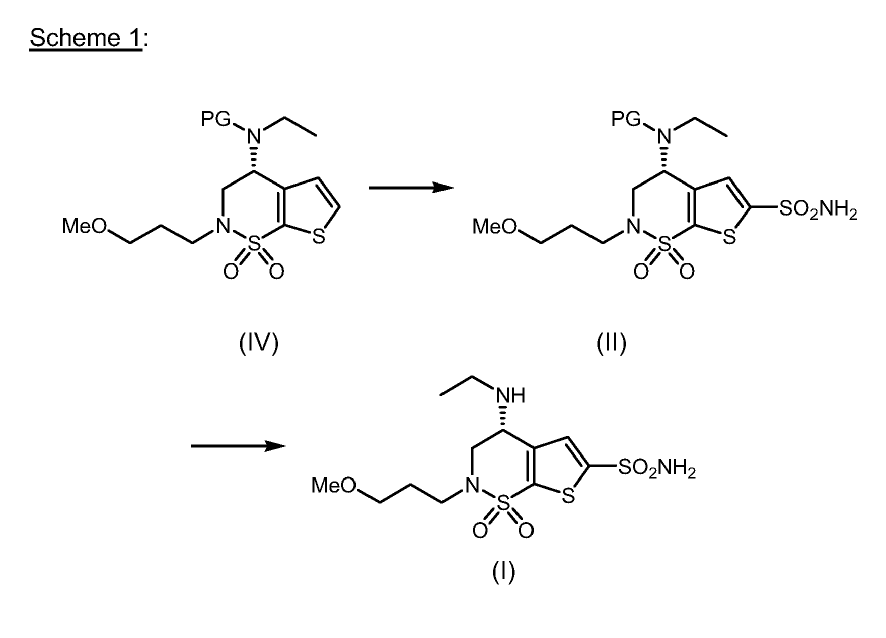

The halosulfonation reaction is preferably carried out at temperatures of about 0 °C to about the boiling point of the reaction mixture, most preferably at about room temperature (i.e., about 25 °C). Preferably, the compound of formula (IV) is added to an excess of halosulfonic acid with stirring and external cooling, usually at 0 °C or, if needed, at about 15 °C to 35 °C. If required, the reaction mixture is heated to complete the halosulfonation reaction. The halosulfonation can be carried out in the absence of a solvent in an excess of halosulfonating agent or in an inert solvent/diluent, for example a halogenated hydrocarbon, ether, alkylnitrile or a mineral acid.

The halosulfonating agent is usually employed in an excess of 1 to 5 times the molar amount, based on the starting material. If the reaction is carried out in the absence of inert solvent, it may be advantageous to use an even greater excess.

Suitable halosulfonating agents are halosulfonic acids such as chlorosulfonic acid, bromosulfonic acid, or fluorosulfonic acid.

Although the complete conversion of the intermediate arylsulfonic acid compound to the desired arylsulfonyl halide can be achieved using a molar excess of halosulfonic acid, in a preferred embodiment, the halosulfonation reaction is carried out using a mixture of an halosulfonating agent and a halogenating agent. By the use of a halogenating agent to carry out the halosulfonation reaction, undesirably intermediates with sulfonic acid groups, which are non-reactive with amidating agents, are avoided, and, consequently higher yields of the desired compound of formula (II) are obtained.

Suitable halogenating agents are phosphoryl halides such as POCl₃ or POBr₃, phosphorus halides such as PCl₅, PBr₅, PCl₃ or PBr₃, or inorganic acid halides such as, for example, thionyl chloride, sulfuryl chloride, N-chlorosuccinimide (NCS), or N-bromosuccinimide (NBS). If appropriate, it may be advantageous to carry out the reaction in the presence of a base, such as, for example, trimethylamine or triethylamine or hexamethyldisilazane. For work-up, the reaction mixture is mixed, for example, with water, and the product can then be isolated as usual.

In the above process, the halosulfonation is preferably a chlorosulfonation, and thus, in a prefered embodiment the halosulfonating agent is a chlorosulfonating agent and the halogenating agent is a chlorinating agent. More preferably, chlorination of sulfonic acid groups is effected using thionyl chloride as the chlorinating agent. Thus, in a more preferred embodiment the chlorosulfonation reaction is carried out with chlorosulfonic acid as chlorosulfonating agent and thionyl chloride as chlorinating agent.

Thionyl chloride is usually added in several fold molar excess to the compound of formula (IV). The temperature at which the desired arylsulfonyl chloride is obtained ranges from about 30 °C to about 80 °C, preferably from about 40 °C to about 60 °C.

After carrying out the halosulfonation reaction, the subsequent amination of the corresponding arylsulfonyl halide intermediate to obtain the compound of formula (II) is performed by reaction with an ammonia source, such as ammonia gas, ammonium hydroxide, or ammonia dissolved in an organic solvent like methanol or dioxane.

The arylsulfonyl halide intermediate obtained after the halosulfonation reaction, i.e. compound of formula (III) 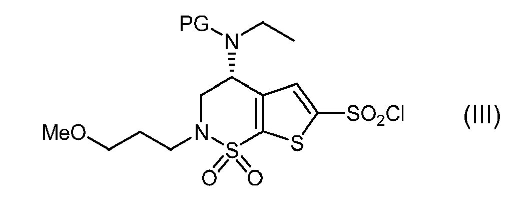 can be isolated before performing the amination reaction. Alternatively, the compound of formula (II) can be obtained from the compound of formula (IV) by carrying out a one pot synthesis without workup of the intermediate products. Avoiding a lengthy separation process and purification of the intermediate chemical compounds, time and resources are saved while increasing chemical yield.

By carrying out the halosulfonation reaction and the subsequent amination of the obtained arylsulfonyl halide intermediate following the process of the present invention, the compound of formula (II) is obtained with better yields, at the same time that more workable temperatures are used, than with the prior art processes. Thus, the reaction can be carried out at 45 °C, which compares favourably with the very low temperatures needed when using BuLi/SO₂, usually between -60 °C and -78°C.

The overall yield provided by the process of sulfonamidation of the position 6 of the 3,4-dihydro-2H-thieno[3,2-e]-1,2-thiazine ring system is also better than the one achieved in US 5378703.

The compound of formula (IV) can be obtained by a protection reaction of the secondary amine of a compound of formula (V) 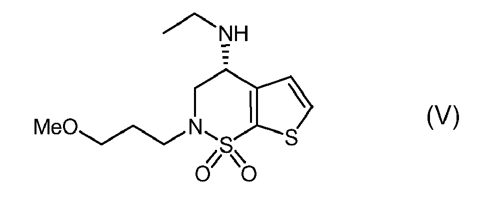 Examples of protecting groups for amino are given in T. W. Greene and P. G. M. Wuts, Protective Groups in Organic Synthesis, (Wiley, 3rd ed. 1999). Among the representative protecting groups for amino those in which the amino group is acylated, such as in the form of an N-alkyl or an N-arylamide, or converted to a carbamate are included. In a preferred embodiment in the compound of formula (IV) the protecting group is an acyl group. When the protecting group is an acyl, preferably, the deprotection is carried out by acid hydrolysis.

The compound of formula (V) can be obtained through the resolution of the corresponding racemate of formula of formula (V')

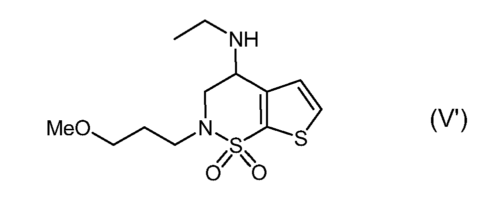

Preferably, the resolution will be carried out with an optically active acid.

Generally weak carboxylic acids are preferred. Suitable optically active carboxylic acid resolving agents include tartaric acid, diacetyl tartaric acid, dibenzoyl tartaric acid, di-p-toluyl tartaric acid, malic acid, camphoric acid, menthoxyacetic or mandelic acid. More preferably the optically active acid is (-)-di-p-toluyl-tartaric acid. The solvent(s) will have to be properly chosen to effectuate the precipitation of the diastereomeric salt of the compound of formula (V) as is well known to those skilled in the art.

The compound of formula (V) can also be prepared from the compound of formula (VI) 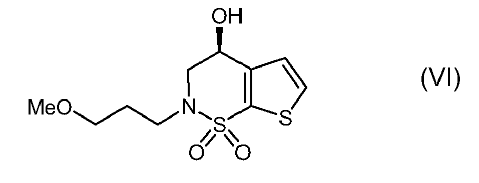 by activation of the hydroxyl group and displacement of the activated hydroxyl group with ethylamine, with inversion of the stereochemistry at C(4).

The activation of the hydroxyl group can be performed by reaction with a sulfonation agent in the presence of an base. In a preferred embodiment the alcohol of formula (VI) is reacted with a sulfonation agent selected from methanesulfonic anhydride and a sulfonyl chloride of formula Cl-SO₂-R₃, wherein R₃ is a radical selected from (C₁-C₄)-alkyl, CF₃, phenyl, and phenyl mono- or disubstituted by a radical selected from (C₁-C₄)-alkyl, bromo and nitro, in the presence of an base, to give the compound of formula (VII).

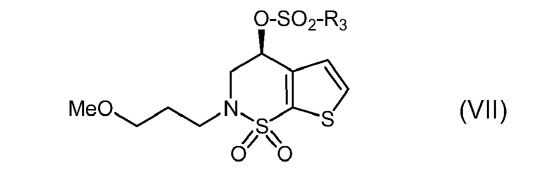

Preferably, R3 is a p-toluenesulfonyl radical.

The sulfonation agent includes methanesulfonyl chloride, p-toluenesulfonyl chloride, benzenesulfonyl chloride, p-bromotoluenesulfonyl chloride, p-nitrobenzenesulfonyl chloride, and trifluoromethanesulfonyl chloride. The activation base includes triethylamine, pyridine, 4-dimethylaminopyridine, and N-methylmorpholine.

To performe the activation of the hydroxyl group, p-toluenesulfonyl chloride and triethylamine are preferred. When tosylation is completed, the displacement reaction is accomplished by adding the ethylamine to the cold solution. The product is isolated by an acid-base work-up.

The process of the present invention includes single reaction steps of the global process to obtain brinzolamide, as well as the combination of two of more sequential steps of the global process described above, the process being able to be carried out in sequential steps isolating the intermediates obtained, or alternatively, in one pot without isolation of any intermediate compound.

The racemic compound of formula (V') can be prepared by following the process represented in Scheme 2.

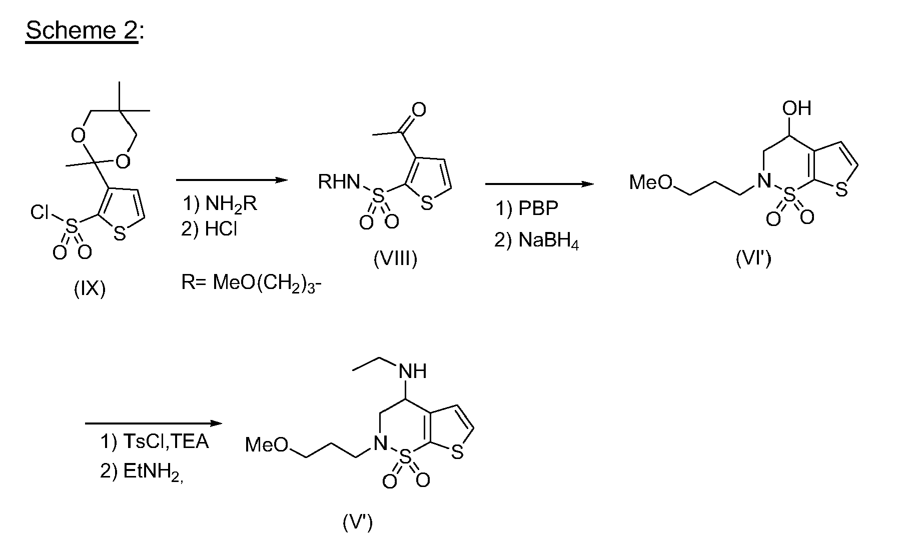

The compound of formula (IX), prepared as disclosed in M.T. Du Priest, et al. "Enantioselective synthesis of AL-4414A, a topically active carbonic anhydrase inhibitor" J. Org. Chem. 1997, vol. 62, pp. 9372-5, is reacted with 3-methoxypropylamine, and then the deketalization to the ketosulfonamide of formula (VIII) is carried out. The introduction of the alkylamine substituent at an earlier step, allows avoiding the reaction of deprotonation (with a strong base such as NaH, dangerous at industrial level) and subsequent alkylation (with the corresponding alkylhalide) carried out in one of the prior art processes. Compound VIII is new and it is also considered part of the present invention. The conversion of this acyclic sulfonamide into the cyclic compound of formula (VI') can be accomplished using a variety of procedures well known in the art, for instance by reaction with pyridinium bromide perbromide (PBP), followed by reaction with sodium borohydride. Finally, the compound of formula V' is obtained as explained above for the compound of formula V.

The preparation of the compound of formula VI can be accomplished by bromination of the compound of formula VIII, for instance with PBP, and the subsequent reduction of the obtained compound of formula X with (+)-β-chlorodiisopinocamphenyl-borane, followed by treatment with aqueous base, as shown in Scheme 3:

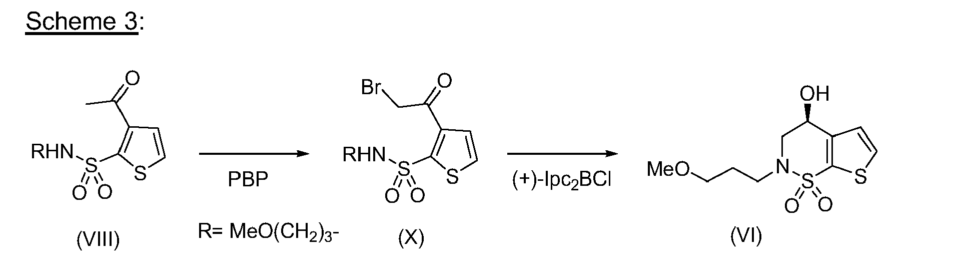

Thus, taking into account all the advantages above mentioned, clearly the alternative process to obtain brinzolamide of the present invention can be considerably more efficient and advantageous than those previously disclosed in the art.

Throughout the description and claims the word "comprise" and variations of the word, such as "comprising", are not intended to exclude other technical features, additives, components, or steps. Additional objects, advantages and features of the invention will become apparent to those skilled in the art upon examination of the description or may be learned by practice of the invention. The following examples and drawings are provided by way of illustration, and are not intended to be limiting of the present invention.

### EXAMPLES

### Example 1: Preparation of N-(3-methoxypropyl)-3-acetyl-2-thiophenesulfonamide (VIII)

To a solution of 3-(2,5,5-trimethyl-1,3-dioxan-2-yl)-2-thiophenesulfonyl chloride (30.75 g, 98.93 mmol) in tetrahydrofuran (175 mL), cooled to 0°C, 3-methoxypropylamine (20 mL, 194.14 mmol) was added slowly. The resulting mixture was heated at 50°C for 1 h. The white solid precipitated was filtered off and the filtrate was concentrated under vacuum to dryness. To a solution of last residue in ketone (170 mL), an aqueous solution of HCl 2N (58 mL) was added. The reaction mixture was heated at reflux until a thin layer chromatography (TLC) showed the absence of starting material. Then, it was concentrated under reduced pressure until obtaining a solid. This residue was cooled at 0°C for 2 h and the white solid was collected by filtration to provide 24.68 g (90% yield) of N-(3-methoxypropyl)- 3-acetyl-2-thiophenesulfonamide.

### Example 2: Preparation of 3,4-dihydro-4-hydroxy-2-(3-methoxy)propyl-2H-thieno[3,2-e]-1,2-thiazine-1,1-dioxide (VI')

To a solution of N-(3-methoxypropyl)-3-acetyl-2-thiophenesulfonamide (21.30 g, 76.79 mmol) in ethyl acetate (370 mL), cooled to 0°C, pyridinium bromide perbromide 90% (24.08 g, 67.76 mmol) and sulphuric acid (9.6 mL) were added slowly. The reaction mixture was stirred during 30 more minutes at 0°C and, after this, water (85 mL) was added. The organic layer was washed with water until the pH of the aqueous phase was pH=3, dried over anhydrous sodium sulphate and concentrated under vacuum. To a solution of the obtained residue in ethanol (210 mL), cooled to 0°C, sodium borohydride (2.90 g, 76.79 mmol) was added slowly. Once the addition was finished, it was allowed to warm to 15°C and stand to this temperature for 1 h. To this crude, NaOH 1 N (150 mL) was added by maintaining the temperature at 15°C. After neutralization with HCl 1N (110 mL) the two phases were separated. The aqueous layer was extracted with ethyl acetate. The combined organic layers were dried over anhydrous sodium sulphate , filtered and concentrated under vacuum to provide 17.68 g (83 % overall yield) of 3,4-dihydro-4-hydroxy-2-(3-methoxy)propyl-2H-thieno[3,2-e]-1,2-thiazine-1, 1-dioxide as an amber oil.

### Example 3: Preparation of 3,4-dihydro-4-(N-ethylamino)-2-(3-methoxy)propyl-2H-thieno[3,2-e]-1,2-thiazine-1,1-dioxide (V')

To a solution of 3,4-dihydro-4-hydroxy-2-(3-methoxy)propyl-2H-thieno[3,2-e]-1,2-thiazine-1,1-dioxide (5.80 g, 20.91 mmol) and triethylamine (6.41 mL, 46.0 mmol) in tetrahydrofuran (80 mL), cooled to 0°C, tosyl chloride (7.97 g, 41.8 mmol) was added. Once the addition was finished, the reaction mixture was allowed to warm to room temperature and stirred overnight. To the resulting suspension, cooled to 0°C, an aqueous solution of ethylamine 70% (49 mL, 627 mmol) was added. Afterwards, the mixture was allowed to warm to room temperature and left overnight. The reaction mixture was acidified with HCl 1N to pH=1-3. After this, ethyl acetate was added and the two phases were separated. The organic phase was washed with HCl 1N and the combined aqueous layers were basified with sodium bicarbonate to pH=8-8.5 and extracted with ethyl acetate. The combined organic layers were dried over anhydrous sodium sulphate , filtered and concentrated under vacuum to provide 4.58 g (72% yield) of 3,4-dihydro-4-(N-ethylamino)-2-(3-methoxy)propyl-2H-thieno[3,2-e]-1,2-thiazine-1,1-dioxide as an amber oil.

### Example 4: Preparation of (R)-3,4-dihydro-4-(N-ethylamino)-2-(3-methoxy)propyl-2H-thieno[3,2-e]-1,2-thiazine-1,1-dioxide (V), di-toluyltartaric salt

A racemic mixture of 3,4-dihydro-4-(N-ethylamino)-2-(3-methoxy)propyl-2H-thieno[3,2-e]-1,2-thiazine-1,1-dioxide (5.00 g, 16.42 mmol) in acetone/water (90/10) (100 mL) was heated to 80°C. To this solution (-)-di-p-toluyl-tartaric acid (3.19 g, 8.21 mmol) was added. The resulting mixture was allowed to cool to 0°C and was stirred overnight. The precipitated white solid was collected by filtration to obtain 3.74 g (33% yield) of (R)-3,4-dihydro-4-(N-ethylamino)-2-(3-methoxy)propyl-2H-thieno[3,2-e]-1,2-thiazine-1,1-dioxide, di-toluyltartaric salt.

### Example 5: Preparation of (R)-4-(N-acetyl-N-ethylamino)-3,4-dihydro-2-(3-methoxy)propyl-2H-thieno[3,2-e]-1,2-thiazine-1,1-dioxide (IV, PG = acetyl)

The (R)-3,4-dihydro-4-(N-ethylamino)-2-(3-methoxy)propyl-2H-thieno[3,2-e]-1,2-thiazine-1,1-dioxide, di-toluyltartaric salt (6.3 g, 9.12 mmol) was treated with a solution of sodium bicarbonate and extracted with dichloromethane to isolate the amine base form. To a solution of this crude (9.12 mmol), and triethylamine (1.8 mL, 12.91 mmol) in dicloromethane (54 mL), cooled to 0°C, acetyl chloride (1.0 mL, 14.06 mmol) was added dropwise. The reaction mixture was stirred vigorously until a TLC showed the absence of starting material. After this, it was neutralized with a saturated solution of bicarbonate and the aqueous phase was extracted with dichloromethane. The combined organic layers were dried over anhydrous sodium sulphate and concentrated under vacuum to provide 2.52 g (80% yield) of (R)-4-(N-acetyl-N-ethylamino)-3,4-dihydro-2-(3-methoxy)propyl-2H-thieno[3,2-e]-1,2-thiazine-1,1-dioxide as a pale yellow oil.

### Example 6: Preparation of (R)-4-(N-Acetyl-N-ethylamine)-3,4-dihydro-2-(3-methoxypropyl)-2H-thieno[3,2-e]-1,2-thiazine-6-sulfonamide-1,1-dioxide (II, PG = acetyl)

To chlorosulfonic acid (9.2 mL, 138.41 mmol) cooled to 0°C, (R)-4-(N-acetyl-N-ethylamino)-3,4-dihydro-2-(3-methoxy)propyl-2H-thieno[3,2-e]-1,2-thiazine-1,1-dioxide (4.8 g, 13.85 mmol) was added. The resulting mixture was heated to 50°C for 6 h. After this, thionyl chloride (9.1 mL, 124.75 mmol) was added dropwise. The mixture was heated during 6 h more. Then, it was allowed to cool to room temperature and it was poured over a water/ice mixture. The resulting solid was filtered off and immediately used into the following stage. The obtained solid was added slowly to a solution of tetrahydrofuran (60 mL) and aqueous ammonia 25% (14 mL) cooled to 0°C. This mixture was allowed to warm to room temperature. The resulting crude was concentrated to dryness to obtain 5.07 g (86% yield) of (R)-4-(N-acetyl-N-ethylamine)-3,4-dihydro-2-(3-methoxypropyl)-2H-thieno[3,2-e]-1,2-thiazine-6-sulfonamide-1,1-dioxide. This product was used in next step of the synthesis without further purification.

### Example 7: Preparation of (R)-3,4-dihydro-4-ethylamino-2-(3-methoxy)propyl-2H-thieno[3,2-e]-1,2-thiazine-6-sulfonamide-1,1-dioxide hydrochloride

To (R)-4-(N-acetyl-N-ethylamine)-3,4-dihydro-2-(3-methoxypropyl)-2H-thieno[3,2-e]-1,2-thiazine-6-sulfonamide-1,1-dioxide (5.0 g, 11.75 mmol), water (170 mL) and sulphuric acid (28 mL) was added. The resulting mixture was heated at reflux until a TLC showed the absence of starting material. The crude reaction was cooled to room temperature, neutralized with NaOH 50% and extracted with ethyl acetate. The combined organic layers were acidified with HCl 36% to pH=3-4 and the precipitated white solid was collected by filtration to provide 4.69 g (95% yield) of (R)-3,4-dihydro-4-ethylamino-2-(3-methoxy)propyl-2H-thieno[3,2-e]-1,2-thiazine-6-sulfonamide-1,1-dioxide, hydrochloride.

## Claims

1. A process for the preparation of brinzolamide of formula (I), or a pharmaceutically acceptable salt thereof, or a solvate thereof including a hydrate, 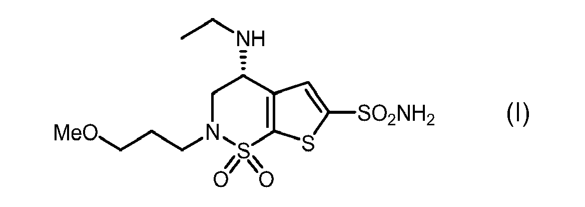 comprising:
a) a halosulfonation reaction of the compound of formula (IV), 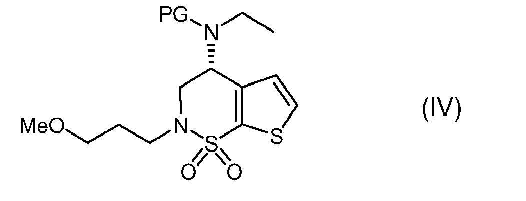 wherein PG is a protecting group, in the presence of a halosulfonating agent, to give a compound of formula (III), 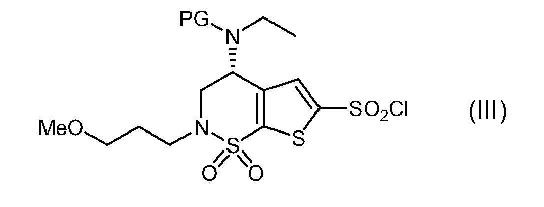 followed by the amination with an ammonia source to give a compound of formula (II), and 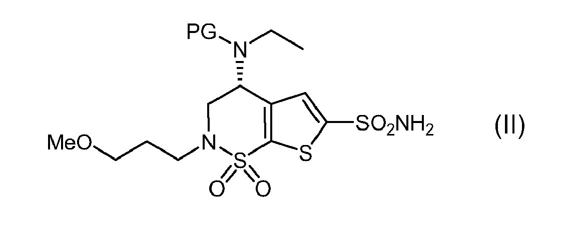
b) a deprotection reaction of the compound of formula (II).

2. The process according to claim 1, wherein the halosulfonation reaction is carried out using a mixture of an halosulfonating agent and a halogenating agent.

3. The process according to claim 2, wherein the halosulfonating agent is a chlorosulfonating agent and the halogenating agent is a chlorinating agent.

4. The process according to claim 3, wherein the chlorosulfonating agent is chlorosulfonic acid and the chlorinating agent is thionyl chloride.

5. The process according to any one of claims 1 to 4, wherein the compound of formula (IV) is obtained by a protection reaction of the secondary amine of a compound of formula (V)

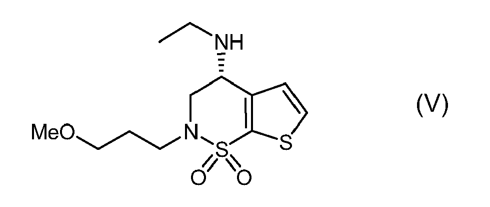

6. The process according to claim 1 to 5, wherein in compound (IV) the protecting group PG is an acyl group.

7. The process according to any one of claims 1 to 6, wherein the compound of formula (V) is previously obtained through the resolution of the corresponding racemate of formula (V').

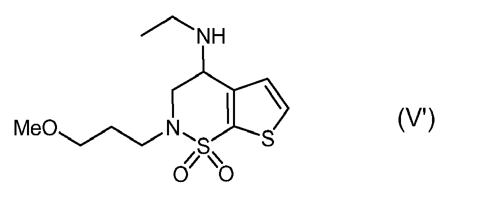

8. The process according to claim 7, wherein the resolution is carried out with (-)-di-p-toluyl-tartaric acid.

9. The process according to any one of claims 1 to 6, wherein the compound of formula (V) is previously prepared by activation of the hydroxyl group of a compound of formula (VI) 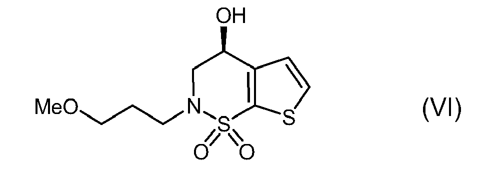 and displacement with ethylamine.

10. The process according to claim 9, wherein the alcohol of formula (VI) is reacted with a sulfonating agent selected from methanesulfonic anhydride and a sulfonyl chloride of formula Cl-SO₂-R₃, wherein R₃ is a radical selected from the group consisting of (C₁-C₄)-alkyl, CF₃, phenyl, and phenyl mono- or disubstituted by a radical selected from the group consisting of (C₁-C₄)-alkyl, bromo and nitro, in the presence of a base, to give a compound of formula (VII).

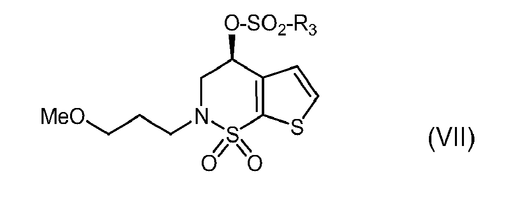

11. A racemic compound of formula (V') or its (R)-stereoisomer of formula (V) 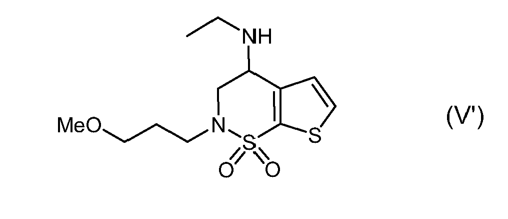 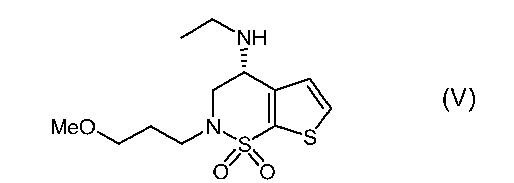 or a pharmaceutically acceptable salt thereof, or a solvate thereof including a hydrate.

12. A compound of formula (IV) 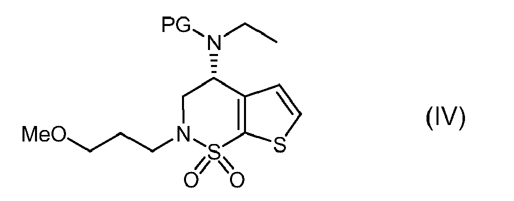 wherein PG is a protecting group, or a pharmaceutically acceptable salt thereof, or a solvate thereof including a hydrate.

13. A compound of formula (II) 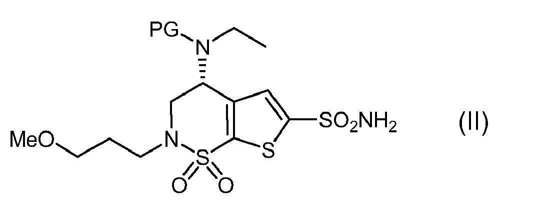 wherein PG is a protecting group, or a pharmaceutically acceptable salt thereof, or a solvate thereof including a hydrate.

14. A compound of formula (III)

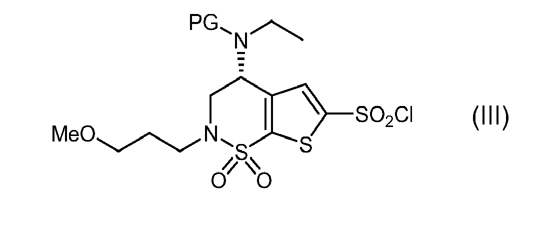

15. A compound of formula (VI)

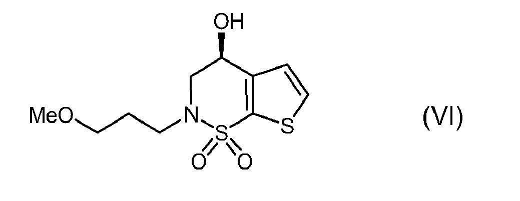

16. A compound of formula (VII)
